(19) Europäisches Patentamt
European Patent Office
Office européen des brevets

(11) Publication number: **0 532 967 B1**

# (12) EUROPEAN PATENT SPECIFICATION

(45) Date of publication of patent specification: **28.06.95**

(51) Int. Cl.$^{6}$: **A61K 7/06**

(21) Application number: **92114844.1**

(22) Date of filing: **31.08.92**

## (54) Hair cosmetic composition.

(30) Priority: **10.09.91 JP 230436/91**

(43) Date of publication of application:
**24.03.93 Bulletin 93/12**

(45) Publication of the grant of the patent:
**28.06.95 Bulletin 95/26**

(84) Designated Contracting States:
**DE FR GB**

(56) References cited:
**EP-A- 0 117 469**
**WO-A-92/17153**
**FR-A- 2 503 561**
**GB-A- 2 148 714**
**US-A- 4 075 131**

**PATENT ABSTRACTS OF JAPAN vol. 13, no. 447 (C-642)(3795)**

(73) Proprietor: **KAO CORPORATION**
**1-14-10, Nihonbashikayaba-cho**
**Chuo-ku**
**Tokyo (JP)**

(72) Inventor: **Sato, Nakako**
**4-7-10-206, Takaido-higashi**
**Suginami-ku,**
**Tokyo (JP)**
Inventor: **Ogawa, Tae**
**1-3-16-404, Higashikoiwa**
**Edogawa-ku,**
**Tokyo (JP)**

(74) Representative: **Hansen, Bernd, Dr. Dipl.-Chem. et al**
**Hoffmann, Eitle & Partner**
**Patent- und Rechtsanwälte,**
**Postfach 81 04 20**
**D-81904 München (DE)**

EP 0 532 967 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to hair cosmetic compositions, and more specifically to hair cosmetic compositions which have excellent hair-setting ability, can provide the tight or stiff feeling and voluminous feeling toward the hair, are neither sticky nor rough to the touch upon applying them to the hair, and hence give users a pleasant feel.

Hair cosmetic compositions such as hair sprays, which are intended for use in setting the hair, generally makes use of an amphoteric polymer having film-forming properties as a setting ingredient. However, these conventional hair cosmetic compositions have involved a drawback that since a great amount of an amphoteric polymer is incorporated to provide a hair-setting effect, the resulting film becomes too hard, and the hair is hence roughened, so that they give users a disagreeable feel.

Accordingly, it has also been attempted to use an oily substance in combination with the amphoteric polymer with a view toward solving this drawback. However, this method is also accompanied by a problem that the hair-setting ability is highly lowered though the feel upon use is improved.

Hydrolyzates of elastin have been known to have an excellent water-retaining effect and have hence been used in hair cosmetic compositions including preparations for preventing the damage to the hair and restoring the damaged hair (Japanese Patent Application Laid-Open Nos. 116204/1984, 186909/1984 and 175924/1989). However, these hair cosmetic compositions containing the hydrolyzates of elastin have involved a problem of insufficient hair-setting ability though they have possessed a good water-retaining effect.

Therefore, there has been a demand for the development of a hair cosmetic composition which is not rough to the touch upon applying it to the hair and hence gives users a pleasant feel upon use, and has excellent hair-setting ability.

In view of the foregoing circumstances, the present inventors have carried out an extensive investigation with a view toward Solving the above-described problems. As a result, it has been found that a hair cosmetic composition making use of an elastin hydrolyzate and an amphoteric polymer having film-forming properties in combination can form a film, which has proper hardness and flexibility, and is hard to destroy, on the surface of the hair, so that it can provide the tight or stiff feeling and the voluminous feeling toward the hair, and moreover has excellent hair-setting ability and gives users an remarkably favorable feel upon applying it to the hair, leading to completion of the present invention.

According to this invention, there is thus provided a hair cosmetic composition comprising an elastin hydrolyzate and an amphoteric polymer having film-forming properties.

The elastin hydrolyzate useful in the practice of this invention can be obtained in accordance with the conventionally-known preparation process. For example, an elastin hydrolyzate obtained by hydrolyzing insoluble elastin extracted from the cutaneous tissue of an animal with a proteolytic enzyme and having a molecular weight of 3,000-300,000 can be used.

In this invention, such an elastin hydrolyzate may be used in the form of either a solution or powder and is commercially available. As examples thereof, may be mentioned "Elasgen Fiber V" (Trademark: a solution containing 15% of elastin peptide) and "Elasgen Powder" (Trademark), both, produced by Ichimaru Pharcos Co., Ltd.

These elastin hydrolyzates are preferably incorporated in a proportion of 0.01-10 wt.% (hereinafter indicated merely by "%"), particularly 0.1-10% based on the whole weight of the hair cosmetic composition according to this invention.

No particular limitation is imposed on the amphoteric polymer having film-forming properties (hereinafter referred to as "amphoteric polymer" simply), which is useful in the practice of this invention, so long as it has film-forming properties. However, particular preference is given to polymers represented by the following general formula (1) or those represented by the following general formula (2) and designated as octylacrylamide/acrylate/butylaminoethyl/methacrylate copolymers in the CFTA dictionary:

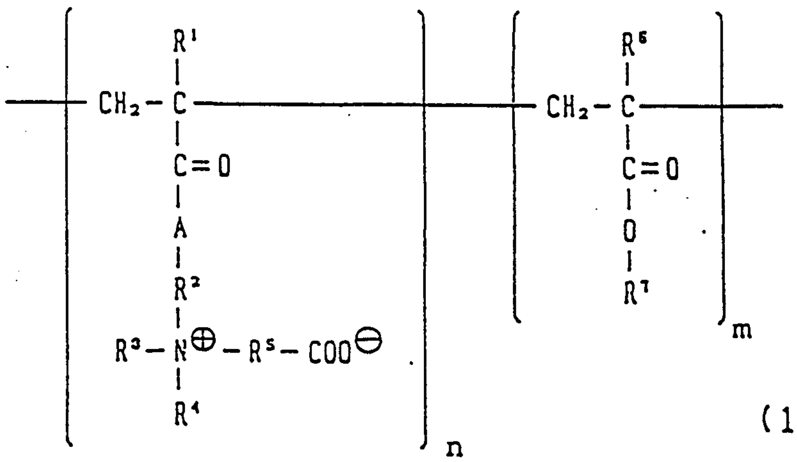

(1)

wherein $R^1$ and $R^6$ mean individually a hydrogen atom or a methyl group, $R^3$ and $R^4$ denote individually an alkyl group having 1-18 carbon atoms, $R^2$ and $R^5$ represent individually an alkylene group having 1-4 carbon atoms, $R^7$ means a saturated or unsaturated hydrocarbon group having 1-24 carbon atoms, A denotes an oxygen atom or an NH group, and n and m stand individually for such a number that a ratio of n to m ranges from 90:10 to 10:90 and the molecular weight of the polymer amounts to a range of 10,000-500,000; or

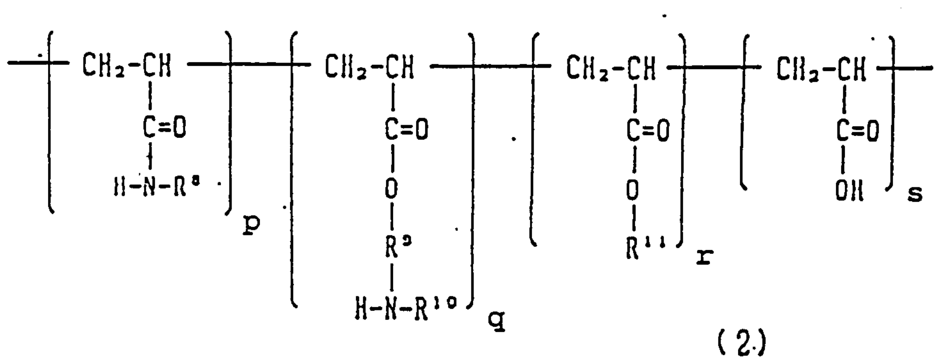

(2)

wherein $R^8$ and $R^{11}$ mean individually an alkyl group having 1-24 carbon atoms, $R^9$ denotes an alkylene group having 1-5 carbon atoms, $R^{10}$ represents an alkyl group having 1-5 carbon atoms, and p, q, r and s stand individually for such a number that the molecular weight of the polymer amounts to a range of 50,000-150,000.

In the formula (1), specific examples of $R^3$ and $R^4$ may include methyl, ethyl, propyl and butyl groups, etc. As specific examples of $R^2$ and $R^5$, may be mentioned methylene, ethylene and propylene groups, etc. As specific examples of $R^7$, may be mentioned butyl, cetyl and stearyl groups, etc.

In the formula (2), specific examples of $R^8$ and $R^{11}$ may include butyl, cetyl and stearyl groups, etc. As specific examples of $R^9$, may be mentioned methylene, ethylene, propylene and pentamethylene groups, etc. As specific examples of $R^{10}$, may be mentioned methyl, ethyl, propyl, butyl and pentyl groups, etc.

Such amphoteric polymers are commercially available. As examples thereof, may be mentioned "Amphomer" (Trademark) produced by National Starch K.K., "Amerset" (Trademark) produced by Amerchol Company, "Amphoset" (Trademark) produced by Mitsubishi Petrochemical Company, Limited, "Yukaformer" (Trademark: containing 30% of an amphoteric polymer) produced by Mitsubishi Petrochemical Company, Limited, and the like.

These amphoteric polymers are preferably incorporated in a proportion of 0.01-30%, particularly 0.1-15% based on the whole weight of the hair cosmetic composition according to this invention.

In this invention, a weight ratio of the elastin hydrolyzate to the amphoteric polymer is preferably within a range of 1:100 - 30:1. Any weight ratios lower than 1:100 result in a hair cosmetic composition which fails to provide the tight or stiff feeling and the voluminous feeling toward the hair and give users a pleasant feel.

On the other hand, any weight ratios higher than 30:1 result in a hair cosmetic composition having insufficient hair-setting ability. It is hence not preferred to use both components at any weight ratios outside the above range. Among the weight ratios within this range, any weight ratios of the elastin hydrolyzate to the amphoteric polymer ranging from 1:100 to 1:2 provide a hair cosmetic composition having excellent hair-setting ability. On the other hand, any weight ratios ranging from 1:1 to 30:1 provide a hair cosmetic composition far excellent in the provision of the tight or stiff feeling and voluminous feeling toward the hair. The weight ratio may be suitably selected as necessary for the intended application of the hair cosmetic composition.

Further, in the hair cosmetic compositions of this invention, optional ingredients, which are mixed routinely in hair cosmetic compositions, may be incorporated in addition to the above-described essential ingredients so far as they do not impede the effects of the inventive hair cosmetic compositions. As examples of such optional ingredients, may be mentioned medicinally-effective ingredients such as antidandruff agents, disinfectants and vitamins; antiseptics such as Parabens; thickeners such as perfluoropolyether and water-soluble polymers; colorants such as dyes and pigments; conditioning agents such as cationic polymers; pearl-like-hue-imparting agents such as glycol esters; hair-setting polymers such as acrylic resin solutions; various kinds of formulated perfume bases; and other ingredients described in ENCYCLOPEDIA OF CONDITIONING RINSE INGREDIENTS, Micelle Press (1987).

The cosmetic compositions according to this invention can be formulated by mixing the elastin hydrolyzate and amphoteric polymer as the essential ingredients and optionally other ingredients in accordance with a method known per se in the art. They may be provided, for example, as hair foams (hair-treating foams, setting foams), hair lotions, blow-styling compositions, hair jellies and hair rinses. The hair cosmetic compositions of this invention may also be used as mascaras.

The hair cosmetic compositions according to the present invention contain both elastin hydrolyzate and amphoteric polymer having film-forming properties. Accordingly, it is possible to form a film having both proper hardness and flexibility, which has been unable to obtain by either ingredient alone, on the surface of the hair.

Therefore, the hair cosmetic compositions of this invention can provide the tight or stiff feeling and the voluminous feeling toward the hair, and moreover have excellent hair-setting ability, are neither sticky nor rough to the touch upon applying them to the hair and hence give users a remarkably pleasant feel upon use.

The present invention will hereinafter be described more specifically by the following examples. However, it should be borne in mind that this invention is not limited to and by these examples.

Example 1:

A hair foam was formulated by using 90% of a stock solution having the following composition and 10% of LP gas as a propellant and charging them in a pressure container.

| <Composition> | Inventive product 1 | Inventive product 2 |
|---|---|---|
| Ethanol | 10.0 (%) | 10.0 (%) |
| Elastin hydrolyzate solution*1 | 2.0 | 4.0 |
| Amphoteric polymer*2 | 0.6 | 0.6 |
| Stearyltrimethylammonium chloride | 0.1 | 0.1 |
| Polyoxyethylene sec-tetradecyl ether | 0.5 | 0.5 |
| Perfume base | 0.12 | 0.12 |
| Purified water | Balance | Balance |

*1: "Elasgen Fiber V" (Trademark: effective amount of 15%), product of Ichimaru Pharcos Co., Ltd.

*2: Polymer of the general formula (1) in which $R^1$, $R^3$, $R^4$ and $R^6$ = methyl group; $R^2$ = ethylene group; $R^5$ = methylene group; $R^7$ = stearyl group, and A = oxygen atom.

Example 2:

A hair lotion having the following composition was formulated in accordance with the conventionally-known method.

| 〈Composition〉 | | |
|---|---|---|
| | Inventive product 3 | Inventive product 4 |
| Ethanol | 55.0 (%) | 60.0 (%) |
| Elastin hydrolyzate solution*1 | 1.0 | 2.0 |
| Amphoteric polymer*2 | 3.0 | - |
| Amphoteric polymer*3 | - | 0.1 |
| Polyoxyethylene hardened castor oil | 0.2 | 0.2 |
| Perfume base | 0.2 | 0.2 |
| Purified water | Balance | Balance |

*1: "Elasgen Fiber V" (Trademark: effective amount of 15%), product of Ichimaru Pharcos Co., Ltd.
*2: The same polymer as that used in Example 1.
*3: Polymer of the general formula (2) in which $R^8$, $R^{10}$ and $R^{11}$ = methyl group; $R^9$ = methylene group.

Example 3:

A mascara having the following composition was formulated in accordance with the conventionally-known method.

| 〈Composition〉 | |
|---|---|
| | Inventive product 5 |
| Amphoteric polymer*1 | 15.0 (%) |
| Elastin hydrolyzate*2 | 1.0 |
| Butylene glycol | 3.0 |
| Pigment | 10.0 |
| Antiseptic | Proper amount |
| Purified water | Balance |

*1: Polymer of the general formula (1) in which $R^1$, $R^3$, $R^4$ and $R^6$ = methyl group; $R^2$ and $R^5$ = ethylene group; $R^7$ = butyl group, and A = oxygen atom.
*2: "Elasgen Powder"(Trademark), product of Ichimaru Pharcos Co., Ltd.

Example 4:

A blow-styling composition having the following composition was formulated in accordance with the conventionally-known method.

| <Composition> | Inventive product 6 |
|---|---|
| Stearyltrimethylammonium chloride | 0.4 (%) |
| Polyethylene glycol (average molecular weight: 300) | 0.1 |
| Elastin hydrolyzate solution*1 | 2.5 |
| Amphoteric polymer*2 | 0.5 |
| Ethanol | 0.5 |
| Perfume base | 0.3 |
| Purified water | Balance |

*1: "Elasgen Fiber V" (Trademark: effective amount of 15%), product of Ichimaru Pharcos Co., Ltd.

*2: Polymer of the general formula (1) in which $R^1$, $R^3$, $R^4$ and $R^6$ = methyl group; $R^2$ = ethylene group; $R^5$ = methylene group; $R^7$ = ethyl group, and A = oxygen atom.

Example 5

A hair spray was formulated by using 50% of a stock solution having the following composition and 50% of LP gas as a propellant and charging them in a pressure container.

| 〈Composition〉 | |
|---|---|
| | Inventive product 7 |
| Amphoteric polymer*1 | 25.0 (%) |
| Elastin hydrolyzate solution*2 | 1.0 |
| Perfume base | 0.5 |
| Ethanol | Balance |

*1: The same polymer as that used in Example 1.
*2: "Elasgen Fiber V" (Trademark: effective amount of 15%), product of Ichimaru Pharcos Co., Ltd.

Test Example 1:

The inventive product 1 obtained in Example 1 and comparative products 1, 2 and 3 having their corresponding composition shown in Table 1 were evaluated with respect to their hair-setting ability,

susceptibility to deformation of the hair set under high humidity and feel of the hair to the touch (feelings of roughness, stickiness and voluminousness) in accordance with the following evaluation methods and standard. The results are shown in Table 2.

Table 1

(Amount mixed: %)

| | Inventive product 1 | Comp. product 1 | Comp. product 2 | Comp. product 3 |
|---|---|---|---|---|
| Ethanol | 10.0 | 10.0 | 10.0 | 10.0 |
| Elastin hydrolyzate solution (*1) | 2.0 | 2.0 | - | 2.0 |
| Amphoteric polymer (*2) | 0.6 | - | 0.6 | - |
| Anionic polymer (Pectin) | - | - | - | 0.6 |
| Stearyltrimethyl-ammonium chloride | 0.1 | 0.1 | 0.1 | 0.1 |
| Polyoxyethylene sec-tetradecyl ether | 0.5 | 0.5 | 0.5 | 0.5 |
| Perfume base | 0.12 | 0.12 | 0.12 | 0.12 |
| Purified water | Balance | Balance | Balance | Balance |

(*1): "Elasgen Fiber V" (Trademark: effective amount of 15%), product of Ichimaru Pharcos Co., Ltd.

(*2): The same polymer as that used in Example 1.

〈Evaluation methods〉

(1) Set retention:

A hair knot of 18 cm in length and 1.5 g in weight was wetted with water to apply 0.1 g of each of the hair cosmetic compositions to be tested to this hair. The thus-coated hair was wounded around a rod of 2 cm in diameter to air-dry it.

The curled hair taken out of the rod was suspended for 30 minutes in a thermo-hygrostat controlled at 20°C and 65% RH to determine the straightening degree of curl. A hair knot applied with no composition was also treated in the same manner as described above.

(2) Susceptibility to deformation of the hair set (set retention) under high humidity:

The same procedure as described above except that a thermo-hygrostat controlled at 20°C and 98% RH was used was carried out.

(3) Feelings of roughness, stickiness and voluminousness (feel of the hair to the touch):

Two grams of each of the hair cosmetic compositions to be tested were applied to a half area of an experimental model head (wig).

According to a paired comparison test by a panel, the feelings of roughness and stickiness were evaluated by the feel of the hair to the touch with fingers, while the feeling of voluminousness was evaluated visually.

〈Evaluation standard〉

◎: Very good compared with the hair applied with no composition;
○: Good compared with the hair applied with no composition;
△: Comparable to the hair applied with no composition;
x: Poor compared with the hair applied with no composition; and
xx: Very poor compared with the hair applied with no composition.

Table 2

| | Inventive product 1 | Comparative product | | |
|---|---|---|---|---|
| | | 1 | 2 | 3 |
| Hair-setting ability | ◎ | △ | ○ | △ |
| Susceptibility to deformation of the hair set under high humidity | ○ | △ | △ | △ |
| Feeling of roughness | ◎ | ○ | xx | x |
| Feeling of stickiness | ◎ | x | x | x |
| Feeling of voluminousness | ◎ | ○ | xx | △ |

It is understood from the results shown in Table 2 that the hair cosmetic composition according to this invention is excellent in both hair-setting ability and feel upon use..pa

**Claims**

1. A hair cosmetic composition comprising an elastin hydrolyzate and an amphoteric polymer having film-forming properties.

2. The hair cosmetic composition as set forth in claim 1, wherein the elastin hydrolyzate is that obtainable by hydrolyzing insoluble elastin extracted from the cutaneous tissue of an animal with a proteolytic enzyme and having a molecular weight of 3,000-300,000.

3. The hair cosmetic composition as set forth in claim 1, wherein the amphoteric polymer having film-forming properties is represented by the following general formula (1) or (2):

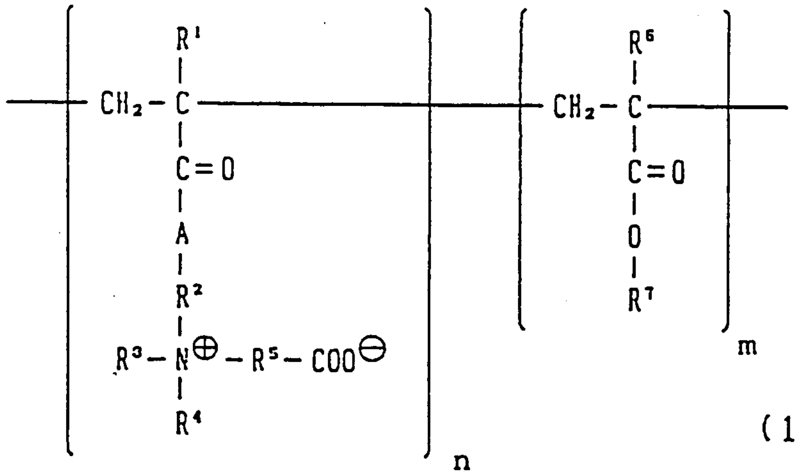

(1)

wherein $R^1$ and $R^6$ mean individually a hydrogen atom or a methyl group, $R^3$ and $R^4$ denote individually an alkyl group having 1-18 carbon atoms, $R^2$ and $R^5$ represent individually an alkylene group having 1-4 carbon atoms, $R^7$ means a saturated or unsaturated hydrocarbon group having 1-24 carbon atoms, A denotes an oxygen atom or an NH group, and n and m stand individually for such a number that a ratio of n to m ranges from 90:10 to 10:90 and the molecular weight of the polymer amounts to a range of 10,000-500,000; or

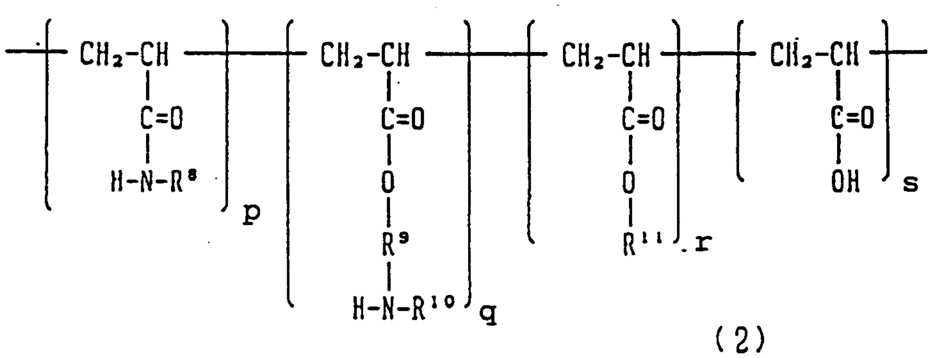

(2)

wherein $R^8$ and $R^{11}$ mean individually an alkyl group having 1-24 carbon atoms, $R^9$ denotes an alkylene group having 1-5 carbon atoms, $R^{10}$ represents an alkyl group having 1-5 carbon atoms, and p, q, r and s stand individually for such a number that the molecular weight of the polymer amounts to a range of 50,000-150,000.

4. The hair cosmetic composition as set forth in claim 1, wherein the elastin hydrolyzate and the amphoteric polymer having film-forming properties are contained in proportions of 0.01-10 wt.% and 0.01-30 wt.%, respectively, based on the whole weight of the hair cosmetic composition.

5. The hair cosmetic composition as set forth in claim 1, wherein a weight ratio of the elastin hydrolyzate to the amphoteric polymer is within a range of 1:100 - 30:1.

6. The hair cosmetic composition as set forth in claim 1, which is suitable for use in setting the hair.

**Patentansprüche**

1. Haarkosmetische Zusammensetzung, umfassend ein Elastinhydrolysat und ein amphoteres Polymer mit filmbildenden Eigenschaften.

2. Haarkosmetische Zusammensetzung gemäss Anspruch 1, in der das Elastinhydrolysat dasjenige ist, das durch Hydrolyse von unlöslichem Elastin, welches mit einem proteolytischen Enzym aus dem Hautgewebe eines Tieres extrahiert wird und ein Molekulargewicht von 3000 bis 300.000 hat, erhältlich ist.

3. Haarkosmetische Zusammensetzung gemäss Anspruch 1, in der das amphotere Polymer mit filmbildenden Eigenschaften eine der folgenden allgemeinen Formeln (1) oder (2) hat:

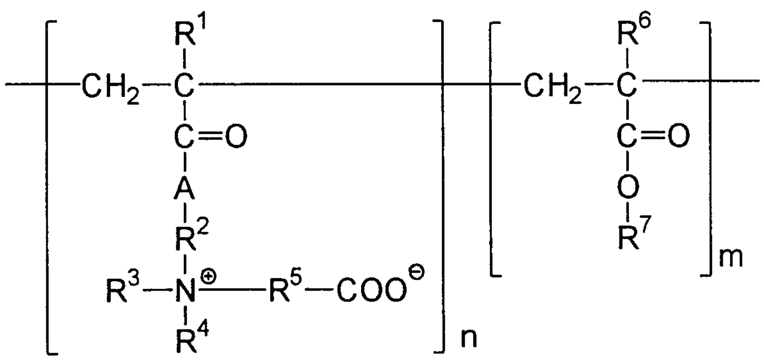

(1)

wobei $R^1$ und $R^6$ jeweils ein Wasserstoffatom oder eine Methylgruppe, $R^3$ und $R^4$ jeweils eine Alkylgruppe mit 1 bis 18 Kohlenstoffatomen, $R^2$ und $R^5$ jeweils eine Alkylengruppe mit 1 bis 4 Kohlenstoffatomen, $R^7$ eine gesättigte oder ungesättigte Kohlenwasserstoffgruppe mit 1 bis 24 Kohlenstoffatomen, A ein Sauerstoffatom oder eine NH-Gruppe bedeuten, und n und m jeweils für eine solche Zahl stehen, dass das Verhältnis von n zu m im Bereich von 90:10 bis 10:90 liegt und das Molekulargewicht des Polymeren 10.000 bis 500.000 beträgt, oder

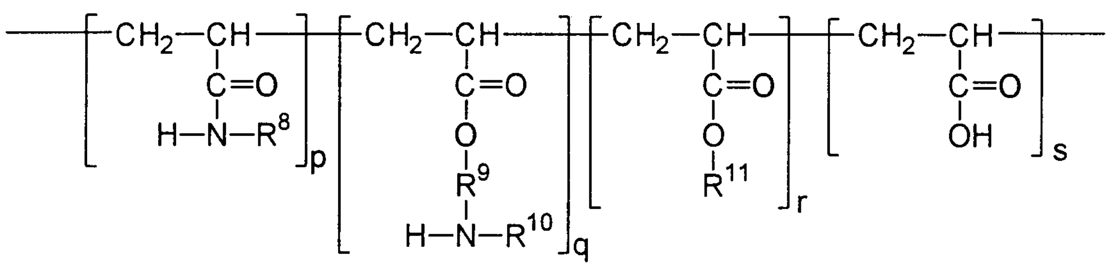

(2)

wobei $R^8$ und $R^{11}$ jeweils eine Alkylgruppe mit 1 bis 24 Kohlenstoffatomen, $R^9$ für eine Alkylengruppe mit 1 bis 5 Kohlenstoffatomen und $R^{10}$ eine Alkylgruppe mit 1 bis 5 Kohlenstoffatomen bedeuten, und p, q, r und s jeweils für solche Zahlen stehen, dass das Molekulargewicht des Polymeren von 50.000 bis 150.000 beträgt.

4. Haarkosmetische Zusammensetzung gemäss Anspruch 1, in der das Elastinhydrolysat und das amphotere Polymer mit filmbildenden Eigenschaften in einer Menge von 0,01 bis 10 Gew.% bzw. 0,01 bis 30 Gew.%, bezogen auf das Gesamtgewicht der haarkosmetischen Zusammensetzung, enthalten sind.

5. Haarkosmetische Zusammensetzung gemäss Anspruch 1, in der das Gewichtsverhältnis von Elastinhydrolysat zu amphoterem Polymer im Bereich von 1:100 bis 30:1 liegt.

6. Haarkosmetische Zusammensetzung gemäss Anspruch 1, die sich zum Festigen von Haaren eignet.

## Revendications

1. Composition cosmétique pour cheveux comprenant un hydrolysat d'élastine et un polymère amphotère ayant des propriétés filmogènes.

2. Composition cosmétique pour cheveux, selon la revendication 1, dans laquelle l'hydrolysat d'élastine est celui pouvant être obtenu en hydrolysant une élastine insoluble extraite du tissu cutané d'un animal avec une enzyme protéolytique et ayant une masse moléculaire de 3 000-300 000.

3. Composition cosmétique pour cheveux selon la revendication 1, dans laquelle le polymère amphotère ayant des propriétés filmogènes est représenté par la formule générale (1) ou (2) suivante :

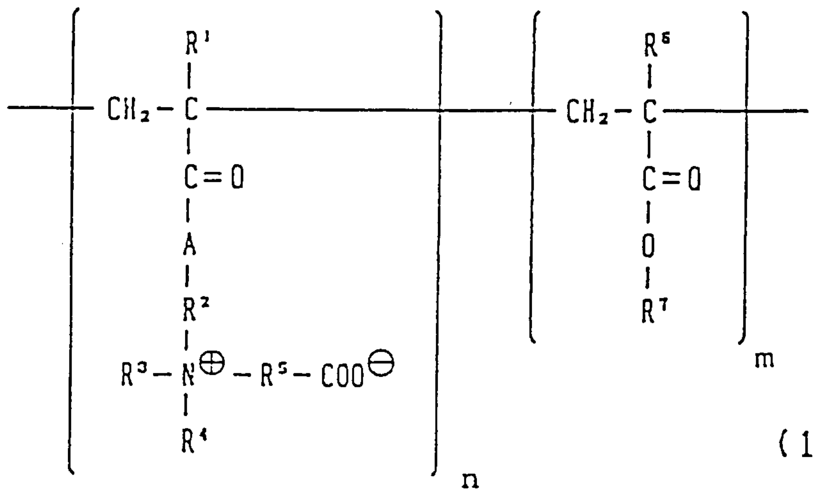

(1)

dans laquelle $R^1$ et $R^6$ sont individuellement un atome d'hydrogène ou un groupe méthyle, $R^3$ et $R^4$ représentent individuellement un groupe alkyle ayant 1-18 atomes de carbone, $R^2$ et $R^5$ représentent individuellement un groupe alkylène ayant 1-4 atomes de carbone, $R^7$ est un groupe hydrocarboné saturé ou insaturé ayant 1-24 atomes de carbone, A représente un atome d'oxygène ou un groupe NH, et n et m représentent individuellement un nombre tel que le rapport de n à m est compris entre 90:10 et 10:90 et la masse moléculaire du polymère s'élève à une valeur dans la gamme 10 000-500 000; ou

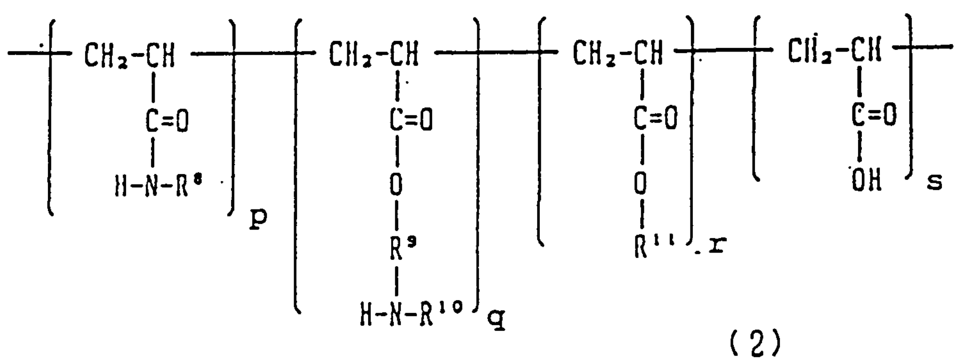

(2)

dans laquelle $R^8$ et $R^{11}$ représentent individuellement un groupe alkyle ayant 1-24 atomes de carbone, $R^9$ représente un groupe alkylène ayant 1-5 atomes de carbone, $R^{10}$ représente un groupe alkyle ayant 1-5 atomes de carbone, et p, q, r et s représentent individuellement un nombre tel que la masse moléculaire du polymère s'élève à une valeur comprise entre 50 000 et 150 000.

**4.** Composition cosmétique pour cheveux selon la revendication 1, dans laquelle l'hydrolysat d'élastine et le polymère amphotère ayant des propriétés filmogènes sont contenus dans des proportions de 0,01-10 % en poids et 0,01-30 % en poids, respectivement, par rapport au poids total de la composition cosmétique pour cheveux.

**5.** Composition cosmétique pour cheveux selon la revendication 1, dans laquelle le rapport pondéral de l'hydrolysat d'élastine au polymère amphotère est dans la gamme de 1:100 - 30:1.

**6.** Composition cosmétique pour cheveux selon la revendication 1, qui convient à la mise en plis des cheveux.